Europäisches Patentamt

European Patent Office

Office européen des brevets

(11) Publication number: **0 086 861**
**B1**

## (12) EUROPEAN PATENT SPECIFICATION

(45) Date of publication of patent specification: **25.09.85**

(51) Int. Cl.⁴: **C 12 N 9/26**

(21) Application number: **82101409.9**

(22) Date of filing: **24.02.82**

(54) **Process for the extraction of beta-amylase from grains.**

(43) Date of publication of application:
**31.08.83 Bulletin 83/35**

(45) Publication of the grant of the patent:
**25.09.85 Bulletin 85/39**

(84) Designated Contracting States:
**AT BE CH DE FR GB LI NL SE**

(56) References cited:
**DE-C- 202 952**
**US-A-3 492 203**
**US-A-4 024 000**

**CHEMICAL ABSTRACTS, vol. 57, no. 13, 24th
December 1962, column 17074g, Columbus,
Ohio, USA R. VERBEEK-WYNDAELE et al.:
"Beta-amylase in barley embryo and
endosperm"
CHEMICAL ABSTRACTS, vol. 77, no. 19, 6th
November 1972, page 125, no. 123218z,
Columbus, Ohio, USA K. VISURI et al.:
"Purification and characterization of crystalline
beta-amylase form barley"
CHEMICAL ABSTRACTS, vol. 89, no. 19, 6th
November 1978, page 297, no. 160133m,
Columbus, Ohio, USA R. SHINKE et al.: "Types
of amylases and their distribution in rice grain"**

(73) Proprietor: **Suomen Nestesokeri Oy
SF-31600 Jokioinen (FI)**

(72) Inventor: **Lehmussaari, Antti
SF-31600 Jokioinen (FI)**
Inventor: **Mansikkamäki, Aino
SF-31600 Jokioinen (FI)**

(74) Representative: **Reitzner, Bruno, Dr. et al
Patentanwälte Dipl.-Ing. R. Splanemann Dr. B.
Reitzner Tal 13
D-8000 München 2 (DE)**

(56) References cited:
**CHEMICAL ABSTRACTS, vol. 35, no. 7, 10th
April 1941, column 2174, no. 5-6, Columbus,
Ohio, USA G. MALCOLM et al.: "The nature of
the liberation of bound barley amylase as
effected by salt solutions"
CHEMICAL ABSTRACTS, vol. 41, no. 1, 10th
January 1947, column 164f-g, Columbus, Ohio,
USA R.H. HOPKINS et al.: "The beta-amylase of
barley"**

EP 0 086 861 B1

# 0 086 861

**Description**

The present invention relates to a process for the preparation of B-amylase by aqueous extraction of grain, particularly of grains of barley.

β-amylase is a starch-hydrolyzing enzyme developed in grain during the growth period and stored herein. The best-known and studied is β-amylase of barley for its major importance in brewing industry. (D. E. Briggs "Barley", Chapman & Hall, London, 1978 Cook "Barley and Malt", Academic Press, London, 1862, J. R. A. Pollock "Brewing Science", Academic Press, London, 1979).

There are two types of β-amylase preparation on the market, those based on grain and bacteria. A difference between them is e.g. the composition of starch hydrolysis products. A hydrolysis catalyzed by bacteria-based β-amylases produces 15—30% of maltotriose and 50—60% of maltose, while grain-based β-amylases produce mainly maltose. Therefore, a starch hydrolyzed by the grain-based β-amylase has increased sweetness and ability to serve as a nutrient of yeast.

The most important applications of β-amylase are found in brewing and starch industries. If there is not available a malt with sufficient enzymatic activities for mashing, the defect can be overcome by the addition of β-amylase. Enzyme additions are necessary when using unmalted grain for mashing. Effecting the hydrolysis of starch with a grain-based β-amylase will result in sweet, maltose-rich products which can be employed as a raw material of beer and in the production of sweets. In the confectionery industry, it is possible to substitute such maltose-rich syrup for some of the saccharose, since the syrup is inherently very sweet indeed.

The above-cited literature, its references and publication DE—AS 1 792 362 diclose a plurality of methods for the separation of β-amylase from grains and components thereof. A common feature of all these methods is that the grain is initially ground or crushed or that the starting material comprises some mechanically separated component of grain, such as bran. From these starting materials β-amylase is extracted with water, a buffer solution etc., whereby the obtained raw extract contains, together with β-amylase, other soluble grain ingredients which leads to a very arduous purification and concentration process to obtain the enzyme.

Since the hull layers of a grain are semi-permeable and since β-amylase is found relatively near the hull, in the subaleurone layer of a grain, studies were initiated to find out whether it is possible to extract β-amylase from a whole grain as it was presumed that, with the grain's own membranes acting as a kind of filter, it would be possible to obtain already at "the raw extract stage" a preparation of substantially improved purity compared to those extracted from flour or grit. It was unexpectedly discovered that 60—80% of the total amount of β-amylase can be extracted from a grain by the process of the invention in such a manner that the extraction is effected from whole or partially hulled grain, the weight of removed hull material being 0—20% of the weight of a grain, the extraction temperature being 20—55°C, and the extraction period exceeding 5 hours.

The amount, purity and extraction period of the β-amylase to be extracted from grain can be controlled by changing the temperature which affects the permeability and rate of diffusion of the grain membranes. Temperature is preferably within the range of from 20 to 55°C.

Another means of control is partial removal of the outermost hull layers of a grain by applying e.g. the technique known from rice hulling, thus obtaining the desired permeability for the grain membranes. A preferred result is achieved by removing circa 10% of the total weight of a grain, preferably barley, as a hull material in such a manner that endosperm remains intact.

In this process, the outermost layers of the grain endosperm serve as a kind of ultrafilter preventing entirely the passage of insoluble impurities into the leaching water and also substantially restricting the amount of soluble compounds. The obtained β-amylase containing extract can be readily concentrated e.g. by ultrafiltration.

It is preferable to use in the extraction some reducing agent (e.g. $SO_2$) for liberating β-amylase from other proteins, this process being known in the literature. The invention will now be illustrated in more detail by means of the following examples.

Example 1

100 g of unhulled barley was leached in 300 ml of 0,5% Na-metabisulphite at 30°C. Activity of leached β-amylase was observed as a function of time (tables). Activity determinations were carried out by modified Bendelow method (Bendelow, W. M. 1963, Journal of Institute of Brewing, No. 69, p. 467—472). The unit applied was a relative unit U (specific activity of ground barley was marked as 100 U/g of protein). All determinations were effected from filtered samples. By a 100% activity was meant the amount of β-amylase which in corresponding conditions dissolves from ground barley in 18 hours.

2

# 0 086 861

| Ground barley | Leaching time (h) | | | | | | |
|---|---|---|---|---|---|---|---|
| | 2 | 4 | 6 | 7 | 12 | 16 | 18 |
| Activity % | 27 | 49 | 71 | 89 | 96 | 99 | 100 |
| Specific activity (U/g protein) | | | | | | | 100 |
| Activity (U/g dry matter in leaching water) | | | | | | | 30 |

| Unhulled barley | Leaching time (h) | | | | | | |
|---|---|---|---|---|---|---|---|
| | 10 | 20 | 30 | 40 | 50 | 60 | 70 |
| Activity % | 8 | 20 | 39 | 51 | 69 | 76 | 78 |
| Specific activity (U/g protein) | | | | | | | 152 |
| Activity (U/g dry matter in leaching water) | | | | | | | 52 |

## Example 2

The test was effected the same way as in Example 1 except that the grains were hulled so that 10% of total weight was removed as hull fraction.

| | Leaching time (h) | | | | | | |
|---|---|---|---|---|---|---|---|
| | 2 | 5 | 10 | 15 | 20 | 25 | 26 |
| Activity % | 7 | 20 | 48 | 65 | 73 | 79 | 80 |
| Specific activity (U/g protein) | | | | | | | 156 |
| Activity (U/g dry matter in leaching water) | | | | | | | 60 |

## Example 3

Grains were hulled so that 20% of total weight was removed as hull fraction. Otherwise the test was effected the same way as in Example 1.

| | Leaching time (h) | | | | | | |
|---|---|---|---|---|---|---|---|
| | 2 | 5 | 10 | 15 | 18 | 20 | 21 |
| Activity % | 12 | 31 | 57 | 72 | 75 | 76 | 77 |
| Specific activity (U/g protein) | | | | | | | 135 |
| Activity (U/g dry matter in leaching water) | | | | | | | 44 |

## Example 4

For studying the effect of temperature on the dissolving rate of β-amylase and on the purity of the solution, the test corresponding to that of Example 1 was run at temperatures 25°C, 40°C and 50°C, respectively. Material used in these tests was barley with hulling degree of 10%.

| | Leaching time (h) | | | | | | | |
|---|---|---|---|---|---|---|---|---|
| | 2 | 5 | 10 | 20 | 22 | 23 | 24 | 26 |
| Activity % 25°C | 4 | 14 | 35 | 70 | 76 | 79 | 82 | 83 |
| 40°C | 7 | 19 | 41 | 69 | 75 | 78 | 78 | 74 |
| 50°C | 11 | 22 | 47 | 70 | 75 | 74 | 70 | |

The following table illustrates the dissolving rate, activity %, specific activity and activity per g dry matter.

3

| | Hulling degree 10 % | | | ground barley |
|---|---|---|---|---|
| Leaching temperature (°C) | 50 | 40 | 25 | 25 |
| Leaching time (h) | 22 | 23 | 26 | 19 |
| Activity % | 75 | 78 | 83 | 100 |
| Specific activity (U/g protein) | 183 | 178 | 156 | 98 |
| Activity (U/g dry matter in leaching water) | 74 | 78 | 65 | 29 |

Thus, it can be noted from examples 1—4 that

— Purity of β-amylase extracted from whole grain is considerably improved compared to that extracted from flour, although the extraction time is longer (example 1)
— by hulling the grains and/or by elevating temperature the extraction time can be shortened and the extracting β-amylase is still of improved purity as compared to the purity of β-amylase extracted from flour (examples 2, 3 and 4)
— in the most favourable conditions it is possible to extract from whole grains 75—83% of that amount of β-amylase which is extractable from flour. In this case the purity of β-amylase is at least 156/98—183/98 or 1.59—1.87 times higher with respect to protein and 65/29—78/29 or 2.24—2.69 times higher with respect to total dry matter (example 4).

Further purification of β-amylase can be effected by applying any conventional technique, such as spray drying, ultrafiltration, precipitation etc. Since the purity of raw extract can be substantially improved by the present process, the above procedures will be facilitated accordingly.

**Claim**

A process for the preparation of β-amylase by aqueous extraction of grain, particularly of grains or barley, characterized in that the extraction is effected from whole or partially hulled grains, the weight of the removed hull material being 0—20% of the weight of a grain, the extraction temperature being 20—55°C, and the extraction time exceeding 5 hours.

**Patentanspruch**

Verfahren zur Gewinnung von β-Amylase durch wäßrige Extraktion von Körnern, insbesondere von Gerstenkörnern, dadurch gekennzeichnet, daß die Extraktion mit ganzen oder teilweise enthülsten Körnern durchgeführt wird, wobei das Gewicht des entfernten Hülsenmaterials 0 bis 20% bis Gewichts eines Korn beträgt, die Extraktionstemperatur bei 20 bis 55°C liegt und die Extraktionszeit mehr als 5 Stunden beträgt.

**Revendication**

Procédé pour la préparation de β-amylase par extraction aqueuse de grain, particulièrement de grains d'orge, caractérisé par le fait que l'on effectue l'extraction en partant de grains entiers ou partiellement mondés, le poids de la matière de pellicule éliminée étant de 0 à 2% du poids d'un grain, le température d'extraction étant de 20 à 55°C et le temps d'extraction dépassant 5 heures.